# EUROPÄISCHE PATENTANMELDUNG

(11) **EP 0 531 650 A2**
(43) Veröffentlichungstag der Anmeldung: **17.03.1993**
(21) Anmeldenummer: 92111547.3
(22) Anmeldetag: 08.07.1992
(51) Int. Cl.: A61K 7/06, A61K 7/075, A61K 7/08

(54) **Haarwaschmittel**

(30) Priorität: 22.08.1991 DE 4127731
(71) Anmelder: GOLDWELL AKTIENGESELLSCHAFT, D-64297 Darmstadt (DE)
(72) Erfinder: Heinz, Dieter, W-6095 Gustavsburg (DE); Hinz, Sabine, W-6102 Pfungstadt (DE)

(57) **Zusammenfassung**

Die vorliegende Erfindung betrifft ein Haarwaschmittel mit verbesserten haarkonditionierenden Eigenschaften, das mindestens ein anionisches Tensid, vorzugsweise im Gemisch mit einem oberflächenaktiven Alkylpolyglykosid im Verhältnis von 10 : 1 und 1 : 5, sowie ein haarkonditionierendes Wirkstoff-Gemisch enthält, das aus 0,1 bis 1,0 Gew.-% mindestens eines kationischen Polymeren, 0,1 bis 1,0 Gew.-% mindestens eines wasserlöslichen Collagens oder Collagen-Derivats und 0,5 bis 8,0 Gew.-%, jeweils bezogen auf die Gesamtzusammensetzung, mindestens eines Polysiloxans besteht.

## Beschreibung

Die vorliegende Erfindung betrifft ein Haarwaschmittel, das nicht nur eine gründliche und schonende Reinigung des Haares gewährleistet, sondern gleichzeitig auch noch eine haarpflegende, konditionierende Wirksamkeit aufweist.

Haarwaschmittel mit konditionierenden Eigenschaften sind im Prinzip bekannt. So wurde bereits vorgeschlagen, Shampoos auf Basis üblicher Tenside kationische Polymere, beispielsweise quaternäre Cellulosederivate, zuzusetzen, die sich beim Waschvorgang auf dem Haar ablagern und diesem eine verbesserte Kämmbarkeit und einen weichen Griff sowie ein glänzendes Aussehen verleihen sollen.
Auch der Zusatz von flüchtigen und nicht-flüchtigen Silikonen zu Haarwaschmitteln auf Basis anionischer Tenside ist bereits vorgeschlagen worden. Alle diese Shampoos zeigen zwar eine gewisse Wirkung, vermögen jedoch nicht alle an sie gestellten Anforderungen in ausreichendem Maße zu erfüllen.

Es wurde nun gefunden, und das ist Gegenstand der vorliegenden Erfindung, daß ein Haarwaschmittel auf wäßriger Grundlage sowohl eine gute aber gleichzeitig haarschonende Reinigung und eine exzellente haarpflegende Wirkung im Hinblick auf den Griff, die Kämmbarkeit, die Fülle und den Glanz des Haares ausübt, wenn es mindestens ein anionaktives Tensid und darüber hinaus ein Gemisch aus haarkonditionierenden Wirkstoffen enthält, das dadurch gekennzeichnet ist, daß
es aus 0,1 bis 1,0 Gewichtsprozent mindestens eines kationischen Polymeren, 0,1 bis 1,0 Gew.-% mindestens eines wasserlöslichen Collagens oder Collagen-Derivats, und 0,5 bis 8,0 Gewichtprozent mindestens eines Polysiloxans besteht, wobei sich die angegebenen Zahlen jeweils auf die Gesamtzusammensetzung beziehen.

Als im Rahmen der Erfindung geeignete anionische Tenside sind insbesondere die bekannten C₁₀-C₁₈-Alkylethersulfate zu nennen, insbesondere die C₁₂-C₁₄-Alkylethersulfate bzw. Laurylethersulfat.
Ein bevorzugtes Aniontensid besteht aus einem Gemisch aus einem C₁₂-C₁₄-Alkylethersulfat mit beispielsweise 2 bzw. etwa 10 Ethylenoxid-Gruppen im Molekül, und einem α-Olefinsulfonat, insbesondere einem C₁₂-C₁₆-, vorzugsweise C₁₄-C₁₆-Olefinsulfonat, wobei das bevorzugte Gewichtsverhältnis zwischen Alkylethersulfat und α-Olefinsulfonat im Bereich von etwa 5 : 1 bis etwa 3 : 2 liegt.
Es können selbstverständlich auch andere bekannte anionische Tenside allein oder im Gemisch untereinander eingesetzt werden, beispielsweise Alkalisalze von Sulfobernsteinsäurehalbestern, insbesondere das Dinatriumsalz des Monooctylsulfosuccinats und Alkalisalze langkettiger Monoalkylethoxysulfosuccinate.
Weitere geeignete anionaktive Tenside sind Fettalkoholsulfate, beispielsweise Laurylsulfat, Sarkoside, beispielsweise Natriumlaurylsarkosid, Monoglyceridsulfate, Fettsäureamidsulfate, die durch Ethoxylierung und anschließende Sulfatiervng von Fettsäuremonoalkanolamiden hergestellt werden, Tauride, Alkylethercarbonsäuren und schließlich Alkalisalze langkettiger Alkylphosphate, die ebenfalls milde, besonders hautverträgliche Detergentien darstellen. Im Gemisch mit anderen anionischen Tensiden ebenfalls einsetzbar sind EiweißFettsäure-Kondensationsprodukte an sich bekannter Struktur.

Der Anteil der anionaktiven Tenside liegt üblicherweise zwischen etwa 5 und etwa 30, vorzugsweise etwa 8 und etwa 20 Gew.-% der Gesamtzusammensetzung. Eine Übersicht über in Shampoos zum Einsatz gelangende anionaktive Tenside findet sich in der Monographie von K. Schrader, 2. Auflage (1989), Hüthig Buchverlag Heidelberg, S. 683 bis 691.

Gemäß einer bevorzugten Ausführungsform der Erfindung liegt das anionaktive Tensid als Gemisch mit mindestens einem Alkylpolyglykosid der allgemeinen Formel RO(R¹O)ₜZₓ, wobei Z einen reduzierenden Saccharidrest mit 5 bis 6 Kohlenstoffatomen, R eine Alkylgruppe mit 8 bis 18 Kohlenstoffatomen, R¹ einen Ethylen- oder Propylenrest, t null bis zehn und x eins bis fünf bedeuten, wobei das Gewichtsverhältnis von anionischen Tensiden zu Alkylpolyglykosid zwischen 10 : 1 und 1 : 5 liegt, in einer Menge zwischen 10 und 30 Gew.-%, bezogen auf die Gesamtzusammensetzung, vor.

Gemische aus anionaktiven Tensiden und Alkylpolyglykosiden sowie deren Verwendung in Haarwaschmitteln sind an sich bereits bekannt, beispielsweise aus der EP-A 70 074.
Die dort beschriebenen Alkylpolyglykoside sind prinzipiell auch im Rahmen der vorliegenden Erfindung geeignet; allerdings wird erfindungsgemäß als besonders bevorzugtes Alkylpolyglykosid ein solches mit etwa 9 bis 11 Kohlenstoffatomen in der Alkylkette und einem Kondensationsgrad von weniger als 1,4, vorzugsweise etwa 1,35, eingesetzt.
Es ist jedoch selbstverständlich, daß auch andere Alkylpolyglykoside, beispielsweise solche mit 12 bis 18 Kohlenstoffatomen in der Alkylkette und höheren Kondensationsgraden, im Rahmen der Erfindung geeignet sind.

Der Anteil an dem Gemisch aus anionaktivem(n) Tensid(en) und Alkylpolyglykosiden(en) in dem erfindungsgemäßen Haarwaschmittel liegt, wie bereits ausgeführt, zwischen etwa 10 und etwa 30 Gewichtsprozent, bezogen auf die Gesamtzusammensetzung des Haarwaschmittels. Vorzugsweise finden etwa 15 bis 25 Gew.-% dieses Gemisches Einsatz.
Das Gewichtsverhältnis von anionischem(n) Tensid(en) zu Alkylpolyglykosid(en) liegt zwischen 10 : 1 und 1 : 5, vorzugsweise 5 : 1 und 1 : 1, insbesondere bei etwa 2 bis 3 : 1.

Gemische aus Sulfosuccinaten und Alkylpolyglykosiden sind aus der EP-A 358 216 bereits bekannt; die dort beschriebenen Gemische eignen sich vorzüglich zum Einsatz in den erfindungsgemäßen Zusammensetzungen.

Die erfindungsgemäßen Haarwaschmittel können selbstverständlich noch weitere Tenside enthalten, insbesondere nichtionische und amphotere.

Gemäß einer weiteren bevorzugten Ausführungsform der Erfindung enthält das Mittel noch ein oberflächenaktives Aminoxid, vorzugsweise in einer Menge von etwa 1 bis etwa 3 Gew.-% der Gesamtzusammensetzung. Ein bevorzugtes Aminoxid ist beispielsweise das Lauryldimethylaminoxid, jedoch können auch andere Aminoxide im Rahmen der Erfindung eingesetzt werden.

Als weitere nichtionische Tensid-Bestandteile können die erfindungsgemäßen Haarwaschmittel beispielsweise langkettige Fettsäuremono- und -dialkanolamide enthalten, beispielsweise Kokosfettsäuremonoethanolamid oder Myristinfettsäurediethanolamid. Der Anteil dieser Alkanolamide liegt vorzugsweise zwischen etwa 0,2 und 2,5, insbesondere 0,5 und 2 Gewichtsprozent der Gesamtzusammensetzung.
Solche Gemische sind beispielsweise Gegenstand der EP-A 70 076.

Weitere geeignete nichtionische Tenside sind die verschiedenen Sorbitanester, beispielsweise Polyethylenglykolsorbitanstearinsäureester, Fettsäurepolyglykolester oder Fettsäureglyceridoxethylate, Fettalkoholpolyglykolether und auch Mischkondensate aus Ethylenoxid und Propylenoxid, wie sie unter der Handelsbezeichnung "Pluronics" im Verkehr sind. Diese nichtionischen Tenside sind in der Regel in untergeordneten Mengen, d. h. mit weniger als 2,5 Gew.-% der Gesamtzusammensetzung, enthalten.

Schließlich können, falls erwünscht, in den erfindungsgemäßen Haarwaschmitteln auch amphotere Tenside mitverwendet werden. Solche sind insbesondere die verschiedenen bekannten Betaine, wie Fettsäureamidoalkylbetaine, langkettige Alkylaminopropionate sowie Sulfobetaine. Diese Substanzen und ihre Verwendung in Haarwaschmitteln sind ebenfalls bekannt, beispielsweise aus Schrader, l.c., auf den hierzu verwiesen wird. Ihr Anteil in den erfindungsgemäßen Zusammensetzungen liegt regelmäßig unter 5 Gew.-%, vorzugsweise unter 3 Gew.-% der Gesamtzusammensetzung.

Das haarkonditionierende System nach der Erfindung besteht aus drei verschiedenen Bestandteilen.
Essentieller Bestandteil Nr. 1 ist ein kationisches Polymeres in einer Menge von 0,1 bis 1,0, vorzugsweise bis 0,5 und besonders bevorzugt von 0,15 bis 0,4 Gewichtsprozent der Gesamtzusammensetzung.
Aus der EP-A 337 354 ist die Verwendung von kationischen Polymeren mit Alkylpolysaccharid-Tensiden an sich bereits bekannt; jedoch läßt sich daraus nicht auf den synergistischen Effekt dieser Bestandteile in der erfindungsgemäßen Zusammensetzung schließen.

Geeignete kationische Polymere sind beispielsweise kationische Cellulosederivate, wie sie unter der Handelsbezeichnung "Polymer JR" vertrieben werden, quaternisierte Mono- und Copolymere von Diallyldimethylammoniumchlorid, quaternäre Copolymerisate des Vinylpyrrolidons, beispielsweise mit Dimethylaminoethylmethacrylat, Polyaminopolyamide, etc. Es wird hierzu, beispielhaft, auf die Aufzählung in der bereits erwähnten EP-A 337 354, S. 3 bis 7, verwiesen.

Der zweite essentielle Bestandteil des haarkonditionierenden Gemisches im Rahmen der vorliegenden Erfindung ist ein wasserlösliches Collagen bzw. ein Collagen-Derivat, das in den Zusammensetzungen in einer Menge von 0,1 bis 1,0, vorzugsweise 0,2 bis 0,5, insbesondere 0,25 bis 0,35 Gewichtsprozent, bezogen auf die Gesamtzusammensetzung, enthalten ist.
Dabei ist das Gewichtsverhältnis des oben definierten kationischen Polymeren und des Collagens bzw. Collagen-Derivats etwa 2 : 1 bis 1 : 2, insbesondere etwa 1 : 1.
Geeignete wasserlösliche Collagene und Collagen-Derivate sind insbesondere hochreines, weitgehend naturbelassenes Collagen, das unter milden Bedingungen aus Tierhaut, beispielsweise Kalbshaut, extrahiert wurde und eine Tripelhelixstruktur mit Telopeptiden aufweist.
Ebenso geeignet ist ein sogenanntes Atelo-Collagen, das ebenfalls eine Tripelhelixstruktur aufweist, wobei jedoch die Telopeptide entfernt wurden. Ein besonders bevorzugtes Collagen-Derivat ist ein Desamido-Collagen, das durch Alkalibehandlung aus Tierhäuten, insbesondere Kalbshäuten, gewonnen wurde und eine Tripelhelixstruktur aufweist, jedoch partiell desamidiert wurde.
Das Molgewicht dieser Collagene liegt in der Regel im Bereich zwischen etwa 250 000 und 300 000, typischerweise zwischen etwa 270 000 und 290 000.

Schließlich enthält die erfindungsgemäße Zusammensetzung noch 0,5 bis etwa 8 Gew.-%, insbesondere 1 bis 5, besonders bevorzugt 1,5 bis 3 Gew.-% eines Polysiloxans.
Wie bereits ausgeführt, ist die Verwendung von Polysiloxanen als konditionierende Mittel in Haarshampoos im Prinzip bekannt; jedoch läßt sich mit diesen Substanzen alleine, seien es nun leichtflüchtige oder schwerflüchtige cyclische oder lineare Polysiloxane, allein oder im Gemisch untereinander, kein ausreichender Konditioniereffekt in Shampoos erzielen.
Polysiloxane, die sich als Bestandteile in den synergistischen Gemischen nach der Erfindung eignen, sind beispielsweise Dimethyl- oder Methylphenylpolysiloxane, beispielsweise die unter den Trivialnamen bekannten Produkte "Dimethicone" bzw. "Phenyldimethicone", sowie auch flüchtige Siliconöle, beispielsweise Hexamethyldisiloxane oder als Cyclomethicone bekannte Substanzen. Gegebenenfalls können auch polyethermodifizierte Polysiloxane, die zum Teil oberflächenaktive Eigenschaften aufweisen, beispielsweise "Dimethiconecopolyol" bzw. "Cetyldimethiconecopolyol" eingesetzt werden.

Die erfindungsgemäßen Haarwaschmittel können selbstverständlich alle üblichen, in solchen Mitteln zum Einsatz gelangenden Stoffe enthalten. Als solche seien Komplexbildner, Farbstoffe, Konservierungsmittel, pH-Regler, Viskositätsregler, beispielsweise anorganische Salze wie Natriumchlorid oder Natriumsulfat, soweit sie nicht ohnehin in den Tensid-Ausgangsmischungen enthalten sind, Duftstoffe, Perlglanzmittel, Verdickungsmittel, etc. genannt. Eine Auflistung solcher Zusatzstoffe findet sich beispielsweise bei Schrader, l.c., S. 295 - 722.

Die folgenden Beispiele dienen der Illustration der Erfindung.

### Beispiel 1:

| | |
|---|---|
| Natriumlaurylethersulfat (1 - 4 EO-Einheiten; 30%ig) | 20,00 (Gew.-%) |
| C₉ - C₁₁-Alkylpolyglykosid (x = 1,35), 35%ig | 20,00 |
| C₁₄-C₁₆-α-Olefinsulfonat, Natriumsalz (40%ig) | 10,00 |
| Lauryldimethylaminoxid (35%ig) | 4,50 |
| Kokosfettsäure-Eiweiß-Kondensat, Natriumsalz (30%ig) | 2,00 |
| Komplexbildner (HEDP) | 0,50 |
| Trübungsmittel | 2,00 |
| Lösungsvermittler (Polyoxyethylierter hydrierter Rizinusölfettsäureester) | 1,00 |
| Kationisches Cellulosederivat (Polyquaternium-10) | 0,25 |
| Polydimethylsiloxan (Dimethicone) | 2,00 |
| Desamidocollagen (Fa. Merck) | 0,25 |
| Parfum | 0,65 |
| Konservierungsmittel (Parabene) | 0,25 |
| Wasser | @ 100,0 |

In einem Verbrauchertest an zwei Gruppen mit jeweils 10 Probanden wusch sich eine Gruppe einmal täglich mit einem Shampoo der Zusammensetzung nach Beispiel 1 die Haare, die zweite Gruppe benutzte ein Shampoo gleicher Zusammensetzung, jedoch ohne Desamidocollagen.

Nach fünf Tagen wurden die Haare der Probanden verglichen. Es zeigte sich, daß das Haar aller Probanden, die das erfindungsgemäße Shampoo benutzten, einen deutlich verbesserten Glanz aufwies sowie lockerer und glatter wirkte als die Haare der Probanden, die das Shampoo ohne Desamidocollagen angewandt hatten.

### Beispiel 2:

| | |
|---|---|
| Laurylethersulfosuccinat, Dinatriumsalz (2 - 4 EO-Einheiten; 30%ig) | 20,00 (Gew.-%) |
| C₁₄-C₁₆-α-Olefinsulfonat, Natriumsalz (40%ig) | 9,50 |
| C₁₂-C₁₄-Alkylglykosid (x = 1,8)); 35%ig | 18,00 |
| Lauryldimethylaminoxid (35%ig) | 4,00 |
| Sorbitanpolyoxyethylen-160-tristearat | 0,50 |
| C₁₂-C₁₄-Fettsäure-Eiweiß-Kondensat (35%ig) | 1,50 |
| Komplexbildner (EHDP) | 0,50 |
| Trübungsmittel | 1,50 |
| Polydimethyldiallylammoniumchlorid (Polyquaternium-6) | 0,30 |
| Desamidocollagen (Fa. Merck) | 0,25 |
| Dimethylpolysiloxan (Dimethicone) | 2,30 |
| Konservierungsmittel (Parabene) | 0,30 |
| Parfum | 0,70 |
| Phosphorsäure, 85%ig | 0,05 |
| Wasser | @ 100,00 |

Dieses Shampoo verleiht dem Haar Fülle und Glätte und einen lockeren Griff.

### Beispiel 3:

| | |
|---|---|
| Natriumlaurylethersulfat (2 EO-Einheiten; 30%ig) | 18,00 (Gew.-%) |
| Laurylethersulfosuccinat, Dinatriumsalz (30%ig) | 12,00 |
| C₉-C₁₁-Alkylpolyglykosid (x = 1,35); 35%ig | 15,00 |
| Lauryldimethylaminoxid (35%ig) | 6,00 |
| Sorbitanpolyoxyethylen-160-tristearat | 0,50 |
| Kokosfettsäure-Eiweiß-Kondensat, Natriumsalz (30%ig) | 2,20 |
| Komplexbildner (EDTA) | 0,30 |
| Trübungsmittel | 1,50 |
| Polyoxyethylierter hydrierter Rizinusölfettsäureester | 1,00 |
| Quaternäres Copolymerisat aus Vinylpyrrolidon/Dimethylaminoethylmethacrylat (Polyquaternium-11) | 0,35 |
| Desamidocollagen (Fa. Merck) | 0,20 |
| Cyclomethicone/Dimethicone 1 : 1 (Cyclisches und lineares Dimethylpolysiloxan) | 2,50 |
| Parfum | 0,70 |
| Konservierungsmittel (Parabene) | 0,30 |
| Wasser | @ 100,0 |

Dieses Shampoo weist die gleichen positiven Eigenschaften wie diejenigen nach den Beispielen 1 und 2 auf.

### Beispiel 4:

| | |
|---|---|
| Natriumlaurylethersulfat (2 - 4 EO-Einheiten; 30%ig) | 27,5 (Gew.-%) |
| Kokosamidopropylbetain (30%ig) | 5,0 |
| Abietinsäure-Tetrapeptid-Kondensat, Triethanolaminsalz (30%ig) | 5,0 |
| Silikonöl | 2,0 |
| Kationisches Cellulosederivat (Polyquaternium-10) | 0,5 |
| Wasserlösliches Collagen | 0,2 |
| Pflanzenextrakte | 0,2 |
| Perlglanz-Konzentrat (Euperlan^{R} PK 900) | 7,5 |
| Parabene | 0,1 |
| Kochsalz | 1,5 |
| Wasser | @ 100,0 |

### Beispiel 5:

| | |
|---|---|
| Monoethanolaminlaurylsulfat, 30%ig | 30,0 (Gew.-%) |
| Kokosalkyldimethylammoniumbetain (Coco-Betaine); 30%ig | 10,0 |
| Kokosmonoethanolamid, 35%ig | 3,0 |
| Silikonöl | 1,0 |
| Kationisches Cellulosederivat (Polyquaternium-10) | 0,3 |
| Wasserlösliches Collagen | 0,3 |
| Parabene | 0,1 |
| Pflanzenextrakte | 1,0 |
| Wasser | @ 100,0 |

### Beispiel 6:

| | |
|---|---|
| Natriumlaurylethersulfat (2 - 4 EO-Einheiten; 30%ig) | 25,0 (Gew.-%) |
| Laurylethercarbonsäure, Natriumsalz (4 - 5 EO-Einheiten; 90%ig) | 10,0 |
| Silikonöl | 1,5 |
| Desamidocollagen (Fa. Merck) | 0,4 |
| Kationisches Cellulosederivat (Polyquaternium-10) | 0,5 |
| Kräuterextrakte | 0,3 |
| Parabene | 0,1 |
| Schwefelöl | 0,2 |
| Wasser | @ 100,0 |

## Patentansprüche

1. Haarwaschmittel auf wäßriger Grundlage, enthaltend mindestens ein anionaktives Tensid und haarkonditionierende Wirkstoffe, dadurch gekennzeichnet,
daß es als haarkonditionierende Wirkstoffe ein Gemisch aus
a) 0,1 bis 1,0 Gew.-%, bezogen auf die Gesamtzusammensetzung, mindestens eines kationischen Polymeren,
b) 0,1 bis 1,0 Gew.-%, bezogen auf die Gesamtzusammensetzung, mindestens eines wasserlöslichen Collagens oder Collagen-Derivats, sowie
c) 0,5 bis 8,0 Gew.-%, bezogen auf die Gesamtzusammensetzung, mindestens eines Polysiloxans enthält.

2. Haarwaschmittel nach Anspruch 1,
dadurch gekennzeichnet,
daß es als kationisches Polymeres 0,2 bis 0,5 Gew.-%, bezogen auf die Gesamtzusammensetzung, eines quaternären Cellulosederivates enthält.

3. Haarwaschmitel nach Anspruch 1 oder 2,
dadurch gekennzeichnet,
daß es als wasserlösliches Collagenderivat 0,2 bis 0,5 Gew.-%, bezogen auf die Gesamtzusammensetzung, eines Desamidocollagens enthält.

4. Haarwaschmittel nach einem oder mehreren der Ansprüche 1 bis 3,
dadurch gekennzeichnet, daß es 1 bis 4 Gew.-%, bezogen auf die Gesamtzusammensetzung, eines Polysiloxans enthält.

5. Haarwaschmittel nach einem oder mehreren der Ansprüche 1 bis 4, dadurch gekennzeichnet,
daß es das kationische Polymere und das wasserlösliche Collagenderivat im Gewichtsverhältnis von 1:2 bis 2:1 enthält.

6. Haarwaschmittel nach einem der vorhergehenden Ansprüche, daddurch gekennzeichnet,
daß es ein Gemisch aus mindestens einem anionaktiven Tensid und mindestens einem Alkylpolyglykosid der allgemeinen Formel
RO(R¹O)ₜ Zₓ,
wobei Z einen reduzierenden Saccharidrest mit 5 bis 6 Kohlenstoffatomen, R eine Alkylgruppe mit 8 bis 18 Kohlenstoffatomen, R¹ einen Ethylen- oder Propylenrest, t O bis 1O und x 1 bis 5 bedeuten, in einer Menge zwischen 10 und 30 Gew.-%, bezogen auf die Gesamtzusammensetzung, wobei das Gewichtsverhältnis von anionischen Tensiden zu Alkylpolyglykosid zwischen 10:1 und 1:5 liegt, enthält.

7. Haarwaschmittel nach einem oder mehreren der Ansprüche 1 bis 6, dadurch gekennzeichnet,
daß es als anionisches Tensid ein Gemisch aus einem C₁₂-C₁₄-Alkylethersulfat und einem -Olefinsulfonat im Gewichtsverhältnis von 5:1 bis 3:2 enthält.

8. Haarwaschmittel nach einem oder mehreren der Ansprüche 1 bis 6,
dadurch gekennzeichnet,
daß es als anionisches Tensid ein Alkalisalz eines Sulfobernsteinsäurehalbesters enthält.

9. Haarwaschmittel nach einem oder mehreren der Ansprüche 1 bis 8, dadurch gekennzeichnet,
daß es zusätzlich 1 bis 3 Gew.-%, bezogen auf die Gesamtzusammensetzung, eines oberflächenaktiven Aminoxids enthält.

10. Haarwaschmittel nach einem der Ansprüche 6 bis 9,
dadurch gekennzeichnet,
daß es als Alkylpolyglykosid ein C₉-C₁₁-Alkylpolyglykosid mit einem Kondensationsgrad (x) von weniger als 1,4 enthält.
